# EUROPEAN PATENT APPLICATION

(11) **EP 0 639 368 A1**
(43) Date of publication of application: **22.02.1995**
(21) Application number: 93909415.7
(22) Date of filing: 23.04.1993
(51) Int. Cl.: A61K 7/06

(54) **SCALP CARE PRODUCT COMPOSITION**

(30) Priority: 23.04.1992 JP 104664/92; 30.03.1993 JP 72460/93
(71) Applicant: SHISEIDO COMPANY LIMITED, Chuo-ku Tokyo 104 (JP); NIPPON SHINYAKU COMPANY, LIMITED, Minami-ku Kyoto-shi Kyoto 601 (JP)
(72) Inventor: HANZAWA, Chika, The Shiseido Research Center, Yokohama-shi, Kanagawa 223 (JP); UZUKA, Makoto, The Shiseido Research Center, Yokohama-shi, Kanagawa 223 (JP); MATSUDA, Hajime, The Shiseido Research Center, Yokohama-shi, Kanagawa 223 (JP); SAKAI, Tetsuya, Chuo-ku, Tokyo 104 (JP); NISHI, Toyoyuki, Kameoka-shi, Kyoto 621 (JP)
(74) Representative: Rinuy, Santarelli
(86) International application number: JP9300531
(87) International publication number: WO9321895

(57) **Abstract**

A scalp care product composition containing plant extracts for inhibiting the loss of hair of a mammal and promoting the hair growth. Efficacious plant extracts are those of the plants of the family *Annonaceae*, including ones belonging to the genera *Annona* L., *Anomianthus* Sincl., *Ellipeiopsis* R.E. Fries, *Artabotrys* R.Br., *Molodorum* Griff. and *Goniothalamus* Hook.f. In particular, extracts from the leaves, barks, woods, branches, sarcocarps, pericarps, roots or stems of these plants have not only the effects of promoting the hair growth and preventing the loss of hair, but also the effect of preventing dandruff and itch.

## Description

### TECHNICAL FIELD

The present invention relates to a head-treatment composition containing a special plant extract and superior in the effects of prevention of hair loss, promotion of hair growth, etc. in mammals.

### BACKGROUND ART

In the past, there have been known hair tonic compositions containing various types of medicines. For example, vitamins such as vitamin E, amino acids such as methionine, vascodilators such as acetylcholine derivatives, anti-inflammation agents such as extract of Shikon (Lithospermi radix; root of Lithospermum officinale L. subsp. erythrorhizom Hand.-Mazz.) female hormones such as estradiol, skin function promotors such as cepharanthine, etc. have been formulated in and used for the prevention and treatment of hair loss. As those using plant components which can be compared with the present invention, there have been publicized the use of crushed seeds of *Anona Squamosa* L. dispersed in, for example, sesame oil for promoting hair growth (see West German Patent Specification No. 3614448). This plant is probably more accurately *Annona Squamosa* L. The seeds per se, however, are known to exhibit toxicity and therefore, there seems to be a problem for practical use.

Further, conventional hair tonic compositions are considered to be effective for prevention and improvement of, for example, dandruff, ichiness, hair loss, and to promote hair growth and hair health, but none has been obtained which exhibits satisfactory effects.

The object of the present invention is to provide a head-treatment composition which is effective for the prevention of hair loss, is effective for hair growth, etc. and is superior in safety.

### DISCLOSURE OF THE INVENTION

The present inventors have heretofore engaged in intensive studies on the efficacy of various types of plant components in preventing hair loss and promoting hair growth and found that extracts derived from plants belonging to the family *Annonaceae* exhibited the above-mentioned efficacy and were superior in safety. Thus, the present invention has been completed.

In accordance with the present invention, there is provided a head-treatment composition comprising an extract derived from at least one plant belonging to the family *Annonaceae*.

More specifically, there is provided a head-treatment composition comprising an extract derived from one or more plants selected from the group consisting of plants belonging to genuses of the family *Annonaceae* such as the *Annona* L., *Anomianthus* Sincl., *Ellipeiopsis* R.E. Fries, *Artabotrys* R. Br., *Melodorum* Griff., and *Goniothalamus* Hook. f.

Further, in the preferable embodiment of the present invention, there is provided a head-treatment composition comprising an extract derived from a plant of *Annona* *squamosa* L., *Anomianthus dulucis* Sincl., or *Ellipeiopsis* *cherrevensis* R.E. Fries.

The head-treatment composition using the extracts of the above-mentioned plants surprisingly does not exhibit any toxicity, unlike those described in the above-mentioned West German Patent Specification No. 3614448 and not only does it exhibit a superior dandruff preventing effect and hair loss preventing effect or one of the same, but also exhibits a hair growth promoting effect.

### BEST MODE FOR CARRYING OUT THE INVENTION

The plants belonging to the family *Annonaceae* usable in the present invention may be those belonging to any of the genuses so long as they meet with the object of the present invention. As typical examples, mention may be made of *Annona* L., *Anomianthus* Sincl., *Ellipeiopsis* R.E. Fries, *Artabotrys* R. Br., *Melodorum* Griff., *Goniothalamus* Hook. f., etc. Among these, plants belonging to the genus *Annona* L., *Anomianthus* Sincl., and *Ellipeiopsis* R.E. Fries are preferable. Further, while it is not intended to limit the same, in the genus *Annona* L., mention may be made, for example, of *Annona squamosa* L., *A. cherimola* Mill., *A. montana* Macfad., *A. muricata* L., *A. reticulata* L., etc.; in the genus Anomianthus Sincl., *Anomianthus dulucis* Sincl., *A. auritus* Backer ex Heyne, *A. argenteus* Backer, and *A. hetrocarpus* Zoll., and in the genus Ellipeiopsis R.E. Fries, *Ellipeiopsis cherrevensis* R.E. Fries. Further, in the genus *Artabotrys* R. Br., mention may be made of *Artabotrys odaratissima* R.Br. and *Artabotrys harmandii* Finet & Gagnep; in the genus *Melodorum* Griff ., mention may be made of *Melodorum* *aberrans* Sincl.; and in the genus *Goniothalamus* Hook. f., mention may be made of *Goniothalamus tapis* Miq.

The head-treatment composition according to the present invention comprises a component extracted from the constituent parts of the plants of one or a combination of a plurality of these plants. The constituent parts of the plants used in the extraction are not particularly limited, but mention may be made of the leaves, bark, wood, branches, sarcocarp, pericarp, roots, or stems. Note that extracts of the seeds of the *Annonaceae* do not exhibit the toxicity of the seeds themselves, but do not give the desired superior hair growth promoting effect either.

The extraction of the effective ingredient from these constituent parts of plants is performed by drying and powdering these constituent parts, if desired, then using water, lower alkyl alcohols such as methanol, ethanol, propanol, or isopropanol, ketones such as acetone or methylethylketone, esters such as ethyl acetate or butyl acetate, glycols such as ethylene glycol or propylene glycol, alone or in any mixtures of the same. Further, the extraction may be heated, if desired.

The extract thus obtained and used in the present invention may be used directly as a liquid extract, may be used as a concentrated product, or may be used concentrated, dried, and pulverized, depending on the form of preparation of the head-treatment composition containing the same.

The content of the extract in the head-treatment composition according to the present invention is not limited, because the most suitable amount is different depending upon the form of the preparation, but in general the content in the composition may be adjusted to 0.005% by weight to 10% by weight. When the amount is less than 0.005% by weight, the above-mentioned effects in the present invention is not sufficiently exhibited, and when the content is more than 10% by weight is disadvantageous in the compounding of the same.

The head-treatment composition according to the present invention may optionally include, in addition to the above-mentioned essential components, to the extent that the effect of the present invention is not impaired, various components (vehicles or other effective ingredients) generally used in cosmetics, quasi-drugs, pharmaceuticals, etc., for example, oils such as polyoxyethylene (8 mole) oleyl alcohol ether, glyceryl monooleate, blood flow promotors such as nicotinic acid amide, benzyl nicotinate, vitamin E acetate, *Swertia* japonica Makino extract, testosterone-α-reductase inhibitors such as glycyrrhetinic acid, hinokitiol, hormones, such as ethynylestradiol, hair root stimulators such as vitamin H: pantothenylethylether, nonionic surfactants such as sorbitan monolaurate, sorbitan monopalmitate, sorbitan sesquiolate, sorbitan trioleate, polyoxyethylene sorbitan monolaurate, polyoxylethylene sorbitan monostearate, polyethylene glycol monooleate, polyoxyethylene alkyl ether, polyglycol diester, lauryl didiethanol amide, fatty acid isopropanol amide, cationic surfactants such as stearyl trimethyl ammonium chloride, anionic surfactants such as benzalkonium chloride, sodium palmitate, sodium laurate, sodium lauryl sulfate, potassium lauryl sulfate, alkyl sulfuric acid triethanol amine, Turkey Red oil, linear dodecyl benzene sulfonate, polyoxyethylene hardened castor oil, polyoxyethylene hardened castor oil malate, semipolar surfactants such as lauryl dimethyl amine oxide, olein dimethyl amine oxide, ampholytic surfactants, humectants, thickenners, preservatives, antioxidants, flavors, coloring agents, etc.

The head-treatment composition of the present invention thus prepared is not limited in the form of preparation and may be in any form. For example, it may be in the form of a tonic, hair cream, shampoo, scalp treatment, rinse, etc. The methods of preparation of the same may be according to any known methods per se.

The present invention will now be further illustrated by, but is by no means limited to, the following Examples. Note that the amounts of compounding mentioned below are % by weight per preparation.

### Preparation of Extract

### Example 1

Extraction was performed from 210 g of a dried powder of the bark of *Annona squamosa* L. by 0.5 liter of methanol. The extraction was once again performed from the residue by 0.3 liter of methanol. The two extracts were combined and concentrated and dried to a solid under reduced pressure. The amount of the extract obtained was 29 g. The yield was 14%.

### Example 2

Extraction was performed from 10 kg of a dried powder of the bark of *Annona squamosa* L. by 30 liters of ethanol. The extraction was once again performed from the residue by 20 liters of ethanol. The two extracts were combined and concentrated and dried to a solid under reduced pressure. The amount of the extract obtained was 1.2 kg. The yield was 12%.

### Example 3

Extraction was performed from 1.2 kg of a dried powder of the branches of *Anomianthus dulucis* Sincl. by 4 liters of methanol. The extraction was once again performed from the residue by 3 liters of methanol. The two extracts were combined and concentrated and dried to a solid under reduced pressure. The amount of the extract obtained was 94 g. The yield was 8%.

### Example 4

Extraction was performed from 10 kg of a dried powder of the branches of *Anomianthus dulucis* Sincl. by 40 liters of ethanol. The extraction was once again performed from the residue by 30 liters of ethanol. The two extracts were combined and concentrated and dried to a solid under reduced pressure. The amount of the extract obtained was 0.7 kg. The yield was 7%.

### Example 5

Extraction was performed from 1.1 kg of a dried powder of the aerial part of *Ellipeiopsis cherrevensis* R.E. Fries by 4 liters of methanol. The extraction was once again performed from the residue by 3 liters of methanol. The two extracts were combined and concentrated and dried to a solid under reduced pressure. The amount of the extract obtained was 103 g. The yield was 9%.

### Measurement of Hair Promotion Effect (Measurement of Hair Promotion Effect by Mice)

Using male C3H mice (60 days after birth), an experiment was performed according to the method of Ogawa et al. (Normal and Abnormal Epidermal Differentiation, M. Seiji and I.A. Bernstein ed., Todai Shuppankai). The hair on the back of each mouse was shaved off in an area of about 2 x 4 cm and the sample was coated once a day successively every day from the following day. The change of the area of the portion where hair regeneration started was found and a comparison was made of the speed of the hair regeneration. The samples used were obtained by stirring and dissolving the dried product of the various plant extracts in 75% ethanol, adding a surfactant, and dissolving. In the test, use was made of 75% ethanol as a control. For each sample, 10 mice were used and the mean value of the areas was found. The result of the hair regeneration area after 25 days was expressed in terms of a percentage. Note that the amount of compounding of the plant extracts in the preparations are expressed by the ratio of weight using the total volume of the preparation as 100 (that is, weight/volume)

### Examples of Preparation

| Control Example | |
|---|---|
| (1) 75% ethanol | 100 |

| Comparative Example | |
|---|---|
| (1) Methanol extract of seeds of *Annona squamosa* L. | 1.0 |
| (2) 75% ethanol | Balance |

| Preparation Example 1 | |
|---|---|
| (1) Methanol extract of *Annona squamosa* L. bark (dried) | 1.0 |
| (2) 75% ethanol | Balance |

| Preparation Example 2 | |
|---|---|
| (1) Ethanol extract of *Anomianthus dulucis* Sincl. branches (dried) | 0.5 |
| (2) 75% ethanol | Balance |

| Preparation Example 3 | |
|---|---|
| (1) Methanol extract of *Annona squamosa* L. leaves (dried) | 0.5 |
| (2) Acetone extract of *Anomianthus dulucis* Sincl. roots (dried) | 0.2 |
| (3) 75% ethanol | Balance |

| Preparation Example 4 | |
|---|---|
| (1) Methanol extract of *Annona montana* Macfad. roots (dried) | 0.1 |
| (2) Propylene glycol extract of *Annona reticulata* L. rind (dried) | 3 |
| (3) 75% ethanol | Balance |

| Preparation Example 5 | |
|---|---|
| (1) Butylacetate extract of *Annona cherimola* Mill. bark (dried) | 0.05 |
| (2) Acetone extract of *Anomianthus auritus* Backer ex Heyne branches (dried) | 0.7 |
| (3) Hot water extract of *Anomianthus argenteus* Backer fruit (dried) | 0.1 |
| (4) 75% ethanol | Balance |

| Preparation Example 6 | |
|---|---|
| (1) Methanol extract of above ground portion of *Ellipeiopsis cherrevensis* R.E. Fries (dried) | 1.0 |
| (2) 75% ethanol | Balance |

The resultant area of regeneration of hair by the samples of the above comparative Example and preparation Examples (after 25 days) were as follows:

| Sample | Hair Regeneration Area |
|---|---|
| Control | 5.0 (%) |
| Comparative Example | 2.0 |
| Preparation Example 1 | 85.5 |
| Preparation Example 2 | 95.0 |
| Preparation Example 3 | 70.3 |
| Preparation Example 4 | 88.2 |
| Preparation Example 5 | 65.1 |
| Preparation Example 6 | 85.0 |

As is clear from the above results, the significant effect with respect to regeneration of hair was observed in all of the preparation Examples (the present invention).

| Preparation Example 7. Hair Tonic | |
|---|---|
| (1) Ethanol extract of *Annona squamosa* L. bark (dried) | 0.5 |
| (2) Methanol extract of *Anomianthus dulucis* Sincl. branches | 0.1 |
| (3) Propylene glycol | 5.0 |
| (4) Sodium hyaluronate | 0.01 |
| (5) 75% ethanol | Balance |

The effects of the hair tonic produced by the above formulation with regard to dandruff, hair growth, hair loss, and the like were tested through actual usage. Fifteen male subjects (ages 25 to 55) exhibiting dandruff, hair growth, hair loss, and the like were administered the tonic once or twice a day in amounts of 1 to 3 ml at a time over four months. The results of Table 1 were obtained. As is clear from Table 1, the hair tonic exhibited a superior effect with regard to promotion of hair growth and prevention of dandruff and hair loss.

The effects of Table 1 were determined according to the following criteria:

### (1) Dandruff Preventing Effect Test

"Ineffective" means no improvement seen at all despite treatment.

"Effective" means reduction in occurrence of dandruff.

"Ex. effect." (extremely effective) means elimination of occurrence of dandruff.

### (2) Hair Growth Promoting Effect Test

"Ineffective" means no improvement seen at all despite treatment.

"Effective" means new growth of hair observed at over at least 2/3 of hair loss area.

"Ex. effect." (extremely effective) means hair grown over entire hair loss portion.

### (3) Hair Loss Preventing Effect Test

"Ineffective" means no improvement seen at all despite treatment.

"Effective" means reduction in advance of hair loss.

"Ex. effect." (extremely effective) means cessation of hair loss.

**Table 1**

| Results of Test on Actual Use | | | | |
|---|---|---|---|---|
| Subject | Age | Dandruff prevention | Hair growth | Hair loss prevention |
| 1 | 37 | Effective | Effective | Effective |
| 2 | 46 | " | Ex. effect. | " |
| 3 | 47 | " | Effective | Ineffective |
| 4 | 36 | " | " | Effective |
| 5 | 38 | Ineffective | " | " |
| 6 | 25 | Effective | Ineffective | Ineffective |
| 7 | 40 | " | Effective | Effective |
| 8 | 51 | Ex. effect | " | " |
| 9 | 32 | Effective | " | " |
| 10 | 30 | " | " | " |
| 11 | 48 | " | Ex. effect | Ex. effect |
| 12 | 41 | Ineffective | Effective | |
| 13 | 53 | Ex. effect | " | Ex. effect |
| 14 | 49 | Effective | " | Effective |
| 15 | 29 | " | Ineffective | " |

| Preparation Example 8. Hair Tonic | |
|---|---|
| (1) Acetone extract of *Anomianthus auritus* Backer ex Heyne branches (dried) | 0.3 |
| (2) Butyl acetate extract of *Annona cherimola* Mill. bark (dried) | 0.05 |
| (3) Propylene glycol extract of *Annona montana* Macfad. roots (dried) | 1.0 |
| (4) Surfactant | 0.5 |
| (5) Sodium hyaluronate | 0.01 |
| (6) 75% ethanol | Balance |

| Preparation Example 9. Hair Tonic | |
|---|---|
| (1) Methanol extract of *Annona muricata* L. root (dried) | 0.5 |
| (2) Acetone extract of *Annona cherimola* Mill. branches (dried) | 0.03 |
| (3) 1-3 butylene glycol extract of *Anomianthus hetrocarpus* Zoll. leaves (dried) | 2.0 |
| (4) Ethyl acetate extract of *Anomianthus argenteus* Backer bark (dried) | 1.0 |
| (5) Surfactant | 0.3 |
| (6) Propylene glycol | 5.0 |
| (7) Sodium hyaluronate | 0.01 |
| (8) 75% ethanol | Balance |

Both the formulations of Preparation Examples 7 and 8 were shown to be effective for hair growth and prevention of hair loss.

The formulations of Preparation Examples 9 to 14 were produced by the conventional methods.

| Preparation Example 10. Hair Tonic | |
|---|---|
| (1) Hinokitiol | 0.1% |
| (2) *Swertia japonica* Makino extract | 1.0% |
| (3) Vitamin B₆ | 0.2% |
| (4) Vitamin E | 0.01% |
| (5) Menthol | 0.2% |
| (6) Salicylic acid | 0.1% |
| (7) Ethyl acetate extract of *Annona squamosa* L. bark | 0.5% |
| (8) Acetone extract of *Annona cherimola* Mill. branches | 0.3% |
| (9) Methanol extract of *Annona reticulata* L. roots | 0.1% |
| (10) Ethanol extract of *Annona squamosa* L. bark | 0.2% |
| (11) Surfactant | 0.1% |
| (12) Propylene glycol | 2.0% |
| (13) 75% ethanol | Balance |

| Preparation Example 11. Hair Tonic | |
|---|---|
| (1) Hinokitiol | 0.1% |
| (2) *Swertia japonica* extract | 1.0% |
| (3) Vitamin B₆ | 0.2% |
| (4) Vitamin E | 0.01% |
| (5) Menthol | 0.2% |
| (6) Salicylic acid | 0.1% |
| (7) Acetone extract of *Annona montana* Macfad. bark | 1.0% |
| (8) Acetone extract of *Annona squamosa* L. branches | 0.3% |
| (9) Methanol extract of *Annona muricata* L. roots | 0.1% |
| (10) Surfactant | 0.1% |
| (11) Propylene glycol | 2.0% |
| (12) 75% ethanol | Balance |

| Preparation Example 12. Hair Tonic | |
|---|---|
| (1) Hinokitiol | 0.1% |
| (2) *Swertia japonica* Makino extract | 1.0% |
| (3) Vitamin B₆ | 0.2% |
| (4) Vitamin E | 0.01% |
| (5) Menthol | 0.2% |
| (6) Salicylic acid | 0.1% |
| (7) Methanol extract of *Annona squamosa* L. bark | 2.0% |
| (8) Acetone extract of *Annona cherimola* Mill. roots | 0.2% |
| (9) Methanol extract of *Annona reticulata* L. stems | 0.2% |
| (10) Surfactant | 0.1% |
| (11) Propylene glycol | 2.0% |
| (12) 75% ethanol | Balance |

| Preparation Example 13. Hair Tonic | |
|---|---|
| (1) Hinokitiol | 0.1% |
| (2) *Swertia japonica* extract | 1.0% |
| (3) Vitamin B₆ | 0.2% |
| (4) Vitamin E | 0.01% |
| (5) Menthol | 0.2% |
| (6) Salicylic acid | 0.1% |
| (7) Methanol extract of *Anomianthus dulucis* Sincl. bark | 0.1% |
| (8) Ethyl acetate extract of *Anomianthus auritus* Backer ex Heyne bark | 0.05% |
| (9) Methanol extract of aerial part of *Anomianthus argenteus* Backer | 0.1% |
| (10) Acetone extract of *Anomianthus hetrocarpus* Zoll. branches | 0.4% |
| (11) Surfactant | 0.1% |
| (12) Propylene glycol | 2.0% |
| (13) 75% ethanol | Balance |

| Preparation Example 14. Hair Tonic | |
|---|---|
| (1) Hinokitiol | 0.1% |
| (2) *Swertia japonica* extract | 1.0% |
| (3) Vitamin B₆ | 0.2% |
| (4) Vitamin E | 0.01% |
| (5) Menthol | 0.2% |
| (6) Salicylic acid | 0.1% |
| (7) Ethanol extract of *Ellipeiopsis cherrevensis* R.E. Fries. bark | 1.0% |
| (8) Surfactant | 0.1% |
| (9) Propylene glycol | 2.0% |
| (10) 75% ethanol | Balance |

| Preparation Example 15. Hair Tonic | |
|---|---|
| (1) Hinokitiol | 0.1% |
| (2) *Swertia japonica* extract | 1.0% |
| (3) Vitamin B₆ | 0.2% |
| (4) Vitamin E | 0.01% |
| (5) Menthol | 0.2% |
| (6) Salicylic acid | 0.1% |
| (7) Methanol extract of *Melodorum aberrans* Sincl. bark | 1.0% |
| (8) Acetone extract of *Goniothalamus tapis* Miq. roots | 0.2% |
| (9) Methanol extract of *Artabotrys odaratissima* R.Br. stems | 0.2% |
| (10) Methanol extract of aerial part of *Artabotrys harmandii* Finet & Gagnep | 0.1% |
| (11) Surfactant | 0.1% |
| (12) Propylene glycol | 2.0% |
| (13) 75% ethanol | Balance |

The formulations of Preparation Examples 15 to 20 were produced by the conventional methods.

| Preparation Example 16. Shampoo | |
|---|---|
| (1) Sodium coco methyl taurine | 2.0% |
| (2) Polyoxyethylene (8 mole) oleyl alcohol ether | 2.0% |
| (3) Lauric diethanolamide | 4.0% |
| (4) Ethylene glycol fatty acid ester | 1.0% |
| (5) Glycerol | 0.2% |
| (6) Menthol | 0.1% |
| (7) Methanol extract of *Melodorum* aberrans Sincl. bark | 1.0% |
| (8) Acetone extract of *Goniothalamus tapis* Miq. roots | 0.2% |
| (9) Methanol extract of *Artabotrys odaratissima* R.Br. stems | 0.2% |
| (10) Methanol extract of aerial part of *Artabotrys harmandii* Finet & Gagnep | 0.1% |
| (11) Sodium diedetate | 0.1% |
| (12) Flavor | q.s. |
| (13) Purified water | Balance |

| Preparation Example 17. Shampoo | |
|---|---|
| (1) Sodium coco methyl taurine | 2.0% |
| (2) Polyoxyethylene (8 mole) oleyl alcohol ether | 2.0% |
| (3) Lauric diethanolamide | 4.0% |
| (4) Ethylene glycol fatty acid ester | 1.0% |
| (5) Glycerol | 0.2% |
| (6) Menthol | 0.1% |
| (7) Acetone extract of *Ellipeiopsis cherrevensis* R.E. Fries. bark | 1.0% |
| (8) Sodium diedetate | 0.1% |
| (9) Flavor | q.s. |
| (10) Purified water | Balance |

| Preparation Example 18. Shampoo | |
|---|---|
| (1) Sodium coco methyl taurine | 2.0% |
| (2) Polyoxyethylene (8 mole) oleyl alcohol ether | 2.0% |
| (3) Lauric diethanolamide | 4.0% |
| (4) Ethylene glycol fatty acid ester | 1.0% |
| (5) Glycerol | 0.2% |
| (6) Menthol | 0.1% |
| (7) Methanol extract of *Anomianthus dulucis* Sincl. bark | 0.1% |
| (8) Acetic acid ethyl ester extract of *Anomianthus auritus* Backer ex Heyne bark 0.05% | |
| (9) Methanol extract of aerial part of *Anomianthus argenteus* Backer | 0.1% |
| (10) Acetone extract of *Anomianthus hetrocarpus* Zoll. branches | 0.4% |
| (11) *Sodium diedetate* | 0.1% |
| (12) Flavor | q.s. |
| (13) Purified water | Balance |

| Preparation Example 19. Shampoo | |
|---|---|
| (1) Sodium coco methyl taurine | 2.0% |
| (2) Polyoxyethylene (8 mole) oleyl alcohol ether | 2.0% |
| (3) Lauric diethanolamide | 4.0% |
| (4) Ethylene glycol fatty acid ester | 1.0% |
| (5) Glycerol | 0.2% |
| (6) Menthol | 0.1% |
| (7) Ethyl acetate extract of *Annona squamosa* L. bark | 0.5% |
| (8) Acetone extract of *Annona cherimola* Mill. branches | 0.3% |
| (9) Methanol extract of *Annona reticulata* L. roots | 0.1% |
| (10) Ethanol extract of *Annona squamosa* L. bark | 0.2% |
| (11) Sodium diedetate | 0.1% |
| (12) Flavor | q.s. |
| (13) Purified water | Balance |

| Preparation Example 20. Shampoo | |
|---|---|
| (1) Sodium coco methyl taurine | 2.0% |
| (2) Polyoxyethylene (8 mole) oleyl alcohol ether | 2.0% |
| (3) Lauric diethanolamide | 4.0% |
| (4) Ethylene glycol fatty acid ester | 1.0% |
| (5) Glycerol | 0.2% |
| (6) Menthol | 0.1% |
| (7) Acetone extract of *Annona montana* Macfad. bark | 1.0% |
| (8) Acetone extract of *Annona squamosa* L. branches | 0.3% |
| (9) Methanol extract of *Annona muricata* L. roots | 0.1% |
| (10) Sodium diedetate | 0.1% |
| (11) Flavor | q.s. |
| (12) Purified water | Balance |

| Preparation Example 21. Shampoo | |
|---|---|
| (1) Sodium coco methyl taurine | 2.0% |
| (2) Polyoxyethylene (8 mole) oleyl alcohol ether | 2.0% |
| (3) Lauric diethanolamide | 4.0% |
| (4) Ethylene glycol fatty acid ester | 1.0% |
| (5) Glycerol | 0.2% |
| (6) Menthol | 0.1% |
| (7) Methanol extract of *Annona squamosa* L. bark | 2.0% |
| (8) Acetone extract of *Annona cherimola* Mill. roots | 0.2% |
| (9) Methanol extract of *Annona reticulata* L. stems | 0.2% |
| (10) Sodium diedetate | 0.1% |
| (11) Flavor | q.s. |
| (12) Purified water | Balance |

The formulations of Preparation Examples 21 to 26 were produced by the conventional methods.

| Preparation Example 22. Rinse | |
|---|---|
| (1) Stearyl trimethyl ammonium chloride | 1.5% |
| (2) Silicone oil | 3.0% |
| (3) Polyoxyethylene (10 mole) oleyl alcohol ether | 1.0% |
| (4) Glycerol | 5.0% |
| (5) Methanol extract of *Annona squamosa* L. bark | 2.0% |
| (6) Acetone extract of *Annona cherimola* Mill. roots | 0.2% |
| (7) Methanol extract of *Annona reticulata* L. stems | 0.2% |
| (8) Preservative | q.s. |
| (9) UV absorbent | q.s. |
| (10) Purified water | Balance |

| Preparation Example 23. Rinse | |
|---|---|
| (1) Stearyl trimethyl ammonium chloride | 1.5% |
| (2) Silicone oil | 3.0% |
| (3) Polyoxyethylene (10 mole) oleyl alcohol ether | 1.0% |
| (4) Glycerol | 5.0% |
| (5) Methanol extract of *Annona montana* Macfad. bark | 1.0% |
| (6) Acetone extract of *Annona squamosa* L. branches | 0.3% |
| (7) Methanol extract of *Annona muricata* L. roots | 0.1% |
| (8) Preservative | q.s. |
| (9) UV absorbent | q.s. |
| (10) Purified water | Balance |

| Preparation Example 24. Rinse | |
|---|---|
| (1) Stearyl trimethyl ammonium chloride | 1.5% |
| (2) Silicone oil | 3.0% |
| (3) Polyoxyethylene (10 mole) oleyl alcohol ether | 1.0% |
| (4) Glycerol | 5.0% |
| (5) Methanol extract of *Anomianthus dulucis* Sincl. bark | 0.1% |
| (6) Ethyl acetate extract of *Anomianthus auritus* Backer ex Heyne bark | 0.05% |
| (7) Methanol extract of above ground portion of *Anomianthus argenteus* Backer | 0.1% |
| (8) Acetone extract of *Anomianthus hetrocarpus* Zoll. branches | 0.4% |
| (9) Preservative | q.s. |
| (10) UV absorbent | q.s. |
| (11) Purified water | Balance |

| Preparation Example 25. Rinse | |
|---|---|
| (1) Stearyl trimethyl ammonium chloride | 1.5% |
| (2) Silicone oil | 3.0% |
| (3) Polyoxyethylene (10 mole) oleyl alcohol ether | 1.0% |
| (4) Glycerol | 5.0% |
| (5) Methanol extract of *Melodorum* aberrans Sincl. bark | 1.0% |
| (6) Acetone extract of *Goniothalamus tapis* Miq. bark | 0.2% |
| (7) Methanol extract of *Artabotrys odaratissima* R.Br. stems | 0.2% |
| (8) Methanol extract of aerial part of *Artabotrys harmandii* Finet & Gagnep | 0.1% |
| (9) Preservative | q.s. |
| (10) UV absorbent | q.s. |
| (11) Purified water | Balance |

| Preparation Example 26. Rinse | |
|---|---|
| (1) Stearyl trimethyl ammonium chloride | 1.5% |
| (2) Silicone oil | 3.0% |
| (3) Polyoxyethylene (10 mole) oleyl alcohol ether | 1.0% |
| (4) Glycerol | 5.0% |
| (5) Ethyl acetate extract of *Annona squamosa* L. bark | 0.5% |
| (6) Acetone extract of *Annona cherimola* Mill. branches | 0.3% |
| (7) Methanol extract of *Artabotrys reticulata* L. roots | 0.1% |
| (8) Ethanol extract of *Annona squamosa* L. bark | 0.2% |
| (9) Preservative | q.s. |
| (10) UV absorbent | q.s. |
| (11) Purified water | Balance |

| Preparation Example 27. Rinse | |
|---|---|
| (1) Stearyl trimethyl ammonium chloride | 1.5% |
| (2) Silicone oil | 3.0% |
| (3) Polyoxyethylene (10 mole) oleyl alcohol ether | 1.0% |
| (4) Glycerol | 5.0% |
| (5) Acetone extract of *Ellipeiopsis cherrevensis* R.E. Fries bark | 1.0% |
| (6) Preservative | q.s. |
| (7) UV absorbent | q.s. |
| (8) Purified water | Balance |

| Preparation Example 28. Scalp Treatment | |
|---|---|
| (1) Liquid paraffin | 27.0% |
| (2) Stearic acid | 5.0% |
| (3) Cetanol | 5.0% |
| (4) Sorbitan monooleate | 2.0% |
| (5) Polyoxyethylene sorbitan monooleate | 3.0% |
| (6) Methanol extract of *Melodorum* aberrans Sincl. bark | 0.1% |
| (7) Acetone extract of *Goniothalamus tapis* Miq. roots | 0.2% |
| (8) Methanol extract of *Artabotrys odaratissima* R. Br. stems | 0.2% |
| (9) Methanol extract of aerial part of *Artabotrys harmandii* Finet & Gagnep | 0.1% |
| (10) 1,3-Butylene glycol | 5.0% |
| (11) Preservative | q.s. |
| (12) Purified water | Balance |

The components (5) to (9) were dissolved in the components (1) to (4). The result and mixture was heated to 80°C for uniform dissolution, then was cooled to 30°C. This was then mixed and stirred with the components (10) to (12) heated to 30°C.

A similar process of production was used below.

| Preparation Example 29. Scalp Treatment | |
|---|---|
| (1) Liquid paraffin | 27.0% |
| (2) Stearic acid | 5.0% |
| (3) Cetanol | 5.0% |
| (4) Sorbitan monooleate | 2.0% |
| (5) Acetone extract of *Ellipeiopsis cherrevensis* R.E. Fries bark | 1.0% |
| (6) 1,3-Butylene glycol | 5.0% |
| (7) Preservative | q.s. |
| (8) Purified water | Balance |

| Preparation Example 30. Scalp Treatment | |
|---|---|
| (1) Liquid paraffin | 27.0% |
| (2) Stearic acid | 5.0% |
| (3) Cetanol | 5.0% |
| (4) Sorbitan monooleate | 2.0% |
| (5) Polyoxyethylene sorbitan monooleate | 3.0% |
| (6) Methanol extract of *Melodorum* Griff. bark | 0.1% |
| (7) Acetone extract of *Goniothalamus tapis* Miq. roots | 0.2% |
| (8) Methanol extract of *Artabotrys odaratissima* R. Br. stems | 0.2% |
| (9) Methanol extract of aerial part of *Artabotrys harmandii* Finet & Gagnep | 0.1% |
| (10) 1,3-Butylene glycol | 5.0% |
| (11) Preservative | q.s. |
| (12) Purified water | Balance |

| Preparation Example 31. Scalp Treatment | |
|---|---|
| (1) Liquid paraffin | 27.0% |
| (2) Stearic acid | 5.0% |
| (3) Cetanol | 5.0% |
| (4) Sorbitan monooleate | 2.0% |
| (5) Acetone extract of *Ellipeiopsis cherrevensis* R.E. Fries bark | 1.0% |
| (6) 1,3-Butylene glycol | 5.0% |
| (7) Preservative | q.s. |
| (8) Purified water | Balance |

| Preparation Example 32. Scalp Treatment | |
|---|---|
| (1) Liquid paraffin | 27.0% |
| (2) Stearic acid | 5.0% |
| (3) Cetanol | 5.0% |
| (4) Sorbitan monooleate | 2.0% |
| (5) Methanol extract of *Anomianthus dulucis* Sincl. bark | 0.1% |
| (6) Ethyl acetate extract of *Anomianthus auritus* Backer bark | 0.05% |
| (7) Methanol extract of above ground portion of *Anomianthus argenteus* Backer | 0.1% |
| (8) Acetone extract of *Anomianthus hetrocarpus* Zoll. branches | 0.4% |
| (9) 1,3-Butylene glycol | 5.0% |
| (10) Preservative | q.s. |
| (11) Purified water | Balance |

| Preparation Example 33. Scalp Treatment | |
|---|---|
| (1) Liquid paraffin | 27.0% |
| (2) Stearic acid | 5.0% |
| (3) Cetanol | 5.0% |
| (4) Sorbitan monooleate | 2.0% |
| (5) Methanol extract of *Melodorum* aberrans Sincl. bark | 1.0% |
| (6) Acetone extract of *Goniothalamus tapis* Miq. roots | 0.2% |
| (7) Methanol extract of *Artabotrys odaratissima* R. Br. stems | 0.2% |
| (8) Methanol extract of aerial part of *Artabotrys harmandii* Finet & Gagnep | 0.1% |
| (9) 1,3-Butylene glycol | 5.0% |
| (10) Preservative | q.s. |
| (11) Purified water | Balance |

| Preparation Example 34. Scalp Treatment | |
|---|---|
| (1) Liquid paraffin | 27.0% |
| (2) Stearic acid | 5.0% |
| (3) Cetanol | 5.0% |
| (4) Sorbitan monooleate | 2.0% |
| (5) Ethyl acetate extract of *Annona squamosa* L. bark | 0.5% |
| (6) Acetone extract of *Annona cherimola* Mill. branches | 0.3% |
| (7) Methanol extract of *Annona reticulata* L. root | 0.1% |
| (8) Ethanol extract of *Annona squamosa* L. bark | 0.2% |
| (9) 1,3-Butylene glycol | 5.0% |
| (10) Preservative | q.s. |
| (11) Purified water | Balance |

### INDUSTRIAL APPLICABILITY

The present invention provides a head-treatment composition which is effective for the prevention and improvement of dandruff, ichiness, hair loss, etc. and is superior to conventional head-treatment compositions. Further, the head-treatment composition of the present invention can be used as various types of preparations, for example, as a medicinal preparation for treatment for promotion of hair growth and the prevention and improvement of hair loss etc. and further as various types of cosmetics which exhibit an effect of prevention of dandruff and ichiness, in addition to the above effects, and therefore, can be utilized in the medical field and in the manufacture of cosmetics.

## Claims

1. A head-treatment composition comprising an effective amount of an extract derived from at least one plant belonging to the family *Annonaceae* and a vehicle acceptable as an external head-treatment agent.

2. A head-treatment composition as claimed in claim 1, wherein the plant belonging to the family *Annonaceae* is a plant selected from the group consisting of the *Annona* L., *Anomianthus* Sincl., *Ellipeiopsis* R.E. Fries, *Artabotrys* R. Br., *Melodorum* Griff., and *Goniothalamus* Hook. f.

3. A head-treatment composition as claimed in claim 2, wherein the plant is *Annona squamosa* L.

4. A head-treatment composition as claimed in claim 2, wherein the plant is *Anomianthus dulucis* Sincl.

5. A head-treatment composition as claimed in claim 2, wherein the plant is *Ellipeiopsis cherrevensis* R.E. Fries.

6. A head-treatment composition as claimed in claim 1, wherein the vehicle is an aqueous ethanol solution.

7. A head-treatment composition as claimed in claim 1, which is prepared in the form suitable for topical application to the human scalp or hair.

8. A head-treatment composition as claimed in claim 7, wherein the composition is in the form of a hair tonic.

9. A head-treatment composition as claimed in claim 7, wherein the composition is in the form of a shampoo.

10. A head-treatment composition as claimed in claim 7, wherein the composition is in the form of a hair rinse.

11. A head-treatment composition as claimed in claim 7, wherein the composition is in the form of a scalp treatment.
